## Europäisches Patentamt

## European Patent Office

## Office européen des brevets

(11) Veröffentlichungsnummer: **0 131 860**

**B1**

(12) **EUROPÄISCHE PATENTSCHRIFT**

(45) Veröffentlichungstag der Patentschrift:
20.05.87

(51) Int. Cl.⁴: **C 07 C 69/44**

(21) Anmeldenummer: **84107874.4**

(22) Anmeldetag: **05.07.84**

(54) Verfahren zur Herstellung von reinen Adipinsäuremonoestern.

(30) Priorität: **14.07.83 DE 3325372**

(43) Veröffentlichungstag der Anmeldung:
**23.01.85 Patentblatt 85/4**

(45) Bekanntmachung des Hinweises auf die Patenterteilung:
**20.05.87 Patentblatt 87/21**

(84) Benannte Vertragsstaaten:
**BE CH DE FR GB IT LI NL**

(56) Entgegenhaltungen:
**EP-A-0 021 118**
**DE-A-3 317 164**

**CHEMICAL ABSTRACTS, Band 78, Nr. 19, 14. Mai 1973, Columbus, Ohio, USA; A. MATSUDA "Cobalt carbonyl-catalyzed hydroesterification of butadiene with carbon monoxide and methanol", Seite 432, Spalte 2, Abstract Nr. 123957k;**
**CHEMICAL ABSTRACTS Band 80, Nr. 11, 18. März 1974, Columbus, Ohio, USA; Seite 322, Spalte 1, Abstract Nr. 59 502v**

(73) Patentinhaber: **BASF Aktiengesellschaft, Carl-Bosch- Strasse 38, D-6700 Ludwigshafen (DE)**

(72) Erfinder: **Schneider, Heinz- Walter, Dr., Bruesseler Ring 43, D-6700 Ludwigshafen (DE)**
Erfinder: **Richter, Wolfgang, Dr., Osloer Weg 19, D-6700 Ludwigshafen (DE)**
Erfinder: **Disteldorf, Walter, Dr., Portugieser Weg 17, D-6706 Wachenheim (DE)**
Erfinder: **Kummer, Rudolf, Dr., Kreuzstrasse 6, D-6710 Frankenthal (DE)**

## Beschreibung

Bei den bisher bekannten Verfahren zur Herstellung von Adipinsäuremonoestern geht man von Adipinsäure aus und versucht die Veresterung auf der Monoesterstufe zu unterbrechen. Nach dem aus der US–PS–4 314 071 bekannten Verfahren verestert man Adipinsäure mit Alkoholen in wäßriger Lösung in Gegenwart von Schwefelsäure als Katalysator, wobei der Monoester in dem Maße wie er entsteht unverzüglich mit nicht polaren Lösungsmitteln extrahiert wird. Die Umsetzung wird innerhalb von 5 Tagen durchgeführt, was einen erheblichen Zeitaufwand beinhaltet. Darüber hinaus erhält man aus dem Extrakt Adipinsäuremonoester lediglich mit einer Reinheit von 96 %, während es einer komplizierten Aufarbeitung bedarf, um zu reinerem Adipinsäuremonoester zu gelangen.

Auch nach dem in der DE–OS–24 04 359 beschriebenen Verfahren geht man von Adipinsäure aus und verestert diese mit Alkoholen in Gegenwart von mindestens einem Mol Wasser je Mol Adipinsäure. Die Ausbeuten an Adipinsäuremonoester lassen zu wünschen übrig, da als Nebenprodukt erhebliche Mengen an Adipinsäurediester erhalten werden. Letztere müssen unter großem Aufwand abgetrennt werden, um zu reinem Adipinsäuremonoester zu gelangen.

Aus Chemical Abstracts, Vol. 80 (1974) Ref. 59 502v ist auch bekannt, daß man 3-Pentensäureester mit Kohlenmonoxid und Wasserstoff in Gegenwart von Kobaltcarbonylkatalysatoren zu 5-Formylvaleriansäureester umsetzt. Ein Hinweis wie man zu reinen Adipinsäuremonoestern gelangt wird nicht gegeben.

Es war deshalb die technische Aufgabe gestellt, Adipinsäuremonoester in hoher Ausbeute und in hoher Reinheit herzustellen.

Diese technische Aufgabe wird gelöst in einem Verfahren zur Herstellung von reinen Adipinsäuremonoestern, wobei man

a) Pentensäureester mit Kohlenmonoxid und Wasserstoff bei einer Temperatur von 90 bis 140°C unter einem Druck von 5 bis 300 bar in Gegenwart von Carbonylkomplexen des Kobalts oder Rhodiums in an sich bekannter Weise umsetzt und 5-Formylvaleriansäureester abtrennt und

b) die so erhaltenen 5-Formylvaleriansäureester mit molekularem Sauerstoff oder solchen enthaltenden Gasen bei einer Temperatur von 20 bis 100°C unter einem Druck von 1 bis 10 bar oxidiert.

Das neue Verfahren hat den Vorteil, daß es mit hohen Ausbeuten verläuft und zu Adipinsäuremonoester mit einer Reinheit von > 99 % führt.

Als Ausgangsverbindung verwendet man Pentensäureester, z. B. Alkylester mit 1 bis 12 Kohlenstoffatomen, Cycloalkylester mit 5 bis 12 Kohlenstoffatomen, Alkarylester mit 7 bis 9 Kohlenstoffatomen oder Phenylester. Bevorzugt geht man von Pentensäurealkylestern aus, insbesondere solchen, die sich von Alkanolen mit 1 bis 4 Kohlenstoffatomen ableiten. Vorteilhaft verwendet man 4- und/oder 3-Pentensäureester. Geeignete Pentensäureester sind beispielsweise 4-Pentensäuremethylester. 4-Pentensäureethylester, 3-Pentensäurepropylester, 3-Pentensäurebutylester, 4-Pentensäureoctylester, 3-Pentensäurecyclohexylester, 4-Pentensäurebenzylester oder 3-Pentensäurephenylester.

Für die Umsetzung verwendet man ein Gemisch aus Kohlenmonoxid und Wasserstoff. Vorteilhaft enthält das Gemisch Kohlenmonoxid und Wasserstoff im Molverhältnis von 10 : 90 bis 50 : 50. Insbesondere hat sich ein Gemisch im Molverhältnis von Kohlenmonoxid zu Wasserstoff von 10 : 40 bewährt.

Bei der Hydroformylierung wendet man eine Temperatur von 90 bis 140°C und bei Verwendung von Rhodiumcarbonylkomplexen vorteilhaft eine Temperatur von 100 bis 120°C an Ferner hält man einen Druck von 5 bis 300 bar, bei der Verwendung von Rhodiumcarbonylkomplexen vorteilhaft einen Druck von 5 bis 20 bar, ein.

Als Hydroformylierungskatalysatoren verwendet man Carbonylkomplexe des Rhodiums oder Kobalts. Die Carbonylkomplexe können vor der Reaktion hergestellt werden oder bilden sich vorteilhaft in situ aus Salzen von Rhodium oder Kobalt z. B. deren fettsauren Salzen wie Kobaltacetat oder Rhodiumacetat. Vorteilhaft sind die Rhodium- oder Kobaltcarbonylkomplexe zusätzlich durch tertiäre Phosphine oder tertiäre Phosphite modifiziert Geeignet sind beispielsweise tertiäre Alkyl- und Arylphosphine sowie tertiäre Alkyl- und Arylphosphite. Bevorzugt verwendet man Triphenylphosphin, substituierte Triarylphosphine wie Tritolylphosphin, Alkyldiarylphosphine wie Hexyldiphenylphosphin.

Die Rhodiumkonzentration beträgt vorteilhaft 50 bis 500 ppm bezogen auf das Reaktionsgemisch, berechnet als Metall. Die Modifizierung der Carbonylkomplexe des Rhodiums mit den genannten Phosphinen oder Phosphiten hat sich besonders bewährt. Vorteilhaft wendet man Phosphine oder Phosphite in 3- bis 100-fachem molaren Überschuß, bezogen auf Rhodium an.

Falls man Kobaltcarbonylkatalysatoren verwendet, kann man auf die Modifizierung durch Phosphine und Phosphite verzichten, insbesondere dann, wenn man für die Hydroformylierung 3-Pentensäureester verwendet wie sie z. B. direkt bei der Umsetzung von Butadien mit Kohlenmonoxid und Alkanolen in Gegenwart von Kobaltcarbonylkomplexen anfallen. So erhaltene Reaktionsgemische werden vorteilhaft mit Kobaltcarbonylkatalysatoren ohne weitere Zusätze bei einer Temperatur von 100 bis 140°C und unter einem Druck von 150 bis 200 bar hydroformyliert.

Es ist auch möglich, Lösungsmittel mitzuverwenden wie flüssige aromatische Kohlenwasserstoffe z. B. Toluol oder Xylol, ferner Carbonsäureester wie z. B. Essigsäure-, Buttersäure- oder Valeriansäureester sowie hochsiedende Kondensationsprodukte wie sie bei der Reaktion selbst entstehen.

Das bei der Hydroformylierung der Pentenester erhaltene Reaktionsgemisch enthält neben nicht umgesetzten Pentensäureestern, die mitverwendeten Katalysatoren, den als Wertprodukt erzeugten 5-Formylvaleriansäureester, sowie Nebenprodukte wie 4-Formylvaleriansäureester, Valeriansäureester Hydroxycapronsäureester und hochsiedende Kondensationsprodukte. Aus diesen Reaktionsgemischen werden die Reaktionsprodukte zunächst vom Katalysator z. B. durch Destillation oder Extraktion getrennt und danach durch fraktionierte Destillation isoliert. Der so erhaltene 5-Formylvaleriansäureester wird für die Oxidation in der zweiten Stufe verwendet.

Die Oxidation des 5-Formylvaleriansäureesters wird bei einer Temperatur von 20 bis 100°C und unter einem Druck von 1 bis 10 bar mit molekularem Sauerstoff oder molekularem Sauerstoff enthaltenden Gasen durchgeführt. Vorteilhaft hält man hierbei eine Temperatur von 50 bis 80°C ein. Die molekularen Sauerstoff enthaltenden Gase können z. B. bis zu 80 Volumen-% Inerte wie Stickstoff, Kohlendioxid oder Edelgase enthalten. Die Oxidation läuft in der Regel ohne Katalysator ab. Sie kann aber auch durch Zusätze von Katalysatoren wie Alkalihydroxide z. B. Kaliumhydroxyd oder Natriumhydroxyd in Mengen von 0,001 bis 0,5 Gew.-% oder Metallsalze des Kobalts oder Mangans, z. B. Kobaltacetat oder Manganacetat in Mengen von 0,001 bis 0,1 Gew.-%, vorzugsweise 0,02 bis 0,08 Gew.-%, berechnet als Metall, zusätzlich beschleunigt werden.

Durch Destillation des so erhaltenen Reaktionsgemisches erhält man Adipinsäuremonoester der frei von Diester und Adipinsäure ist sowie keine hochsiedenden Anteile enthält.

Adipinsäuremonoester, die nach dem Verfahren der Erfindung erhältlich sind, eignen sich zur Herstellung von Sebacinsäureester nach der Kolbesynthese.

Das Verfahren nach der Erfindung sei an folgendem Beispiel veranschaulicht.

**Beispiel**

In einem Hochdruckgefäß von 2 Liter Inhalt werden 360 g eines Pentensäuremethylestergemisches das 342 g (3 Mol) 4-Pentensäuremethylester enthält, in 600 ml Toluol gelöst. Als Katalysator gibt man 70,2 g (268 mMol) Triphenylphosphin und 108 mg (1,04 mMol) Rhodium in Form der Komplexverbindung $HRhCOL_3$ (L = Triphenylphosphin) zu. Das Reaktionsgemisch wird auf 110°C erhitzt und dann mit einem Gemisch aus 80 Vol.-% Wasserstoff und 20 Vol.-% Kohlenmonoxid auf einen Druck von 8 bar eingestellt. Wenn während der Reaktion der Druck unter 7 bar abfällt wird er durch Nachpressen eines äquimolekularen Gemisches aus Wasserstoff und Kohlenmonoxid wieder auf 8 bar eingestellt. Nach einer Reaktionszeit von 2 h wird die Umsetzung abgebrochen und das Reaktionsgemisch durch Destillation aufgearbeitet. Hierbei erhält man

| | | |
|---|---|---|
| nichtumgesetzten Pentensäuremethylester | 81,3 g | (18,5 %) |
| Valeriansäuremethylester | 9,1 g | ( 2,6 %) |
| 4-Formylvaleriansäuremethylester | 26,8 g | ( 6,2 %) |
| 5-Formylvaleriansäuremethylester | 310,2 g | (71,8 %) |
| Hydroxycapronsäuremethylester | 2,2 g | ( 0,5 %) |
| Hochsiedende Kondensationsprodukte | 1,7 g | ( 0,4 %) |

Die Ausbeute der Hydroformylierung beträgt 71,8 %, die Selektivität zu 5-Formylvalerianester 88,1 %.

288 g (2 Mol) des oben erhaltenen 5-Formylvaleriansäuremethylesters werden in einer Blasensäure während 6 h bei Normaldruck (1 bar) und bei einer Temperatur von 50°C durch Einleiten von Sauerstoff oxidiert. Dabei wird ein Umsatz von > 99 % erzielt. Das erhaltene Reaktionsgemisch wird in einer Füllkörperkolonne bei 2 mbar und einer Temperatur von 126°C destilliert. Man erhält 307 g Adipinsäuremonomethylester in einer Reinheit > 99 %. Die Ausbeute der Oxidation betragt 95,9 %.

**Patentansprüche**

1. Verfahren zur Herstellung von reinen Adipinsäuremonoestern, dadurch gekennzeichnet, daß man
a) Pentensäureester mit Kohlenmonoxid und Wasserstoff bei einer Temperatur von 90 bis 140°C unter einem Druck von 5 bis 300 bar in Gegenwart von Carbonylkomplexen des Kobalts oder des Rhodiums in an sich bekannter Weise umsetzt und 5-Formylvaleriansäureester abtrennt und
b) den so erhaltenen 5-Formylvaleriansäureester mit molekularem Sauerstoff oder solchen enthaltenden Gasen bei einer Temperatur von 20 bis 100°C unter einem Druck von 1 bis 10 bar oxidiert.

2. Verfahren nach Anspruch 1, dadurch gekennzeichnet, daß man 3- und/oder 4-Pentensäure $C_1$- bis $C_4$-Alkylester verwendet.

3. Verfahren nach den Ansprüchen 1 und 2, dadurch gekennzeichnet, daß man Rhodiumcarbonylkomplexe, die mit tertiären Phosphinen oder tertiären Phosphiten modifiziert sind als Katalysatoren verwendet.

4. Verfahren nach den Ansprüche 1 und 2, dadurch gekennzeichnet, daß man 3-Pentensäureester in Gegenwart von Kobaltcarbonylkomplexen umsetzt.

## 0 131 860

### Claims

1. A process for the preparation of a pure monoester of adipic acid, wherein

a) a pentenoate is reacted in a conventional manner with carbon monoxide and hydrogen at a temperature of from 90 to 140°C and under a pressure of from 5 to 300 bar in the presence of a carbonyl complex of cobalt or rhodium, and a 5-formylvalerate is separated off, and

b) the 5-formylvalerate thus obtained is oxidized with molecular oxygen, or with a gas containing this, at a temperature of from 20 to 100°C and under a pressure of from 1 to 10 bar.

2. A process as claimed in claim 1, wherein a $C_1$-$C_4$-alkyl 3-pentenoate or a $C_1$-$C_4$-alkyl 4-pentenoate or a mixture of a $C_1$-$C_4$-alkyl 3-pentenoate and a $C_1$-$C_4$-alkyl 4-pentenoate is used.

3. A process as claimed in claims 1 and 2, wherein a rhodium carbonyl complex modified with a tertiary phosphine or tertiary phosphite is used as the catalyst.

4. A process as claimed in claims 1 and 2, wherein a 3-pentenoate is reacted in the presence of a cobalt carbonyl complex.

### Revendications

1. Procédé de préparation de monoesters d'acide adipique, caractérisé par le fait que:

a) on fait réagir, de manière connue en soi, de l'ester d'acide penténique avec de l'oxyde de carbone et de l'hydrogène, à une température de 90 à 140°C, sous une pression de 5 à 300 bar, en présence de complexes de cobalt-carbonyle ou de rhodium-carbonyle et on sépare l'ester d'acide 5-formylvalérianique et

b) on oxyde l'ester d'acide 5-formylvalérianique ainsi obtenu avec de l'oxygène moléculaire ou des gaz en contenant, à une température de 20 à 100°C, sous une pression de 1 à 10 bar.

2. Procédé selon la revendication 1, caractérisé par le fait qu'on utilise un ester alkylique en $C_1$ à $C_4$ d'acide 3- et/ou 4-penténique.

3. Procédé selon les revendications 1 et 2, caractérisé par le fait qu'on utilise comme catalyseurs des complexes rhodium-carbonyle qui sont modifiés avec des phosphines tertiaires ou des phosphites tertiaires.

4. Procédé selon les revendications 1 et 2, caractérisé par le fait que l'on fait réagir de l'ester d'acide 3-penténique en présence de complexes cobalt-carbonyle.